# EUROPÄISCHE PATENTSCHRIFT

(11) **EP 0 836 538 B1**
(45) Veröffentlichungstag und Bekanntmachung des Hinweises auf die Patenterteilung: **15.09.1999**
(21) Anmeldenummer: 96922023.5
(22) Anmeldetag: 24.06.1996
(51) Int. Cl.: B09B 3/00

(54) **VERFAHREN ZUR ENTSORGUNG VON ABFÄLLEN DER TRANSDERMALEN WIRKSTOFFAPPLIKATION**
WASTE MANAGEMENT PROCESS FOR TRANSDERMAL ACTIVE SUBSTANCES
PROCEDE D'ELIMINATION DE DECHETS DE SUBSTANCES ACTIVES TRANSDERMIQUES

(30) Priorität: 01.07.1995 DE 19524083
(43) Veröffentlichungstag der Anmeldung: 22.04.1998
(73) Patentinhaber: LTS LOHMANN Therapie-Systeme GmbH, 56567 Neuwied (DE)
(72) Erfinder: ASMUSSEN, Bodo, D-56170 Bendorf (DE); HOFFMANN, Hans-Rainer, D-56566 Neuwied (DE); MÜLLER, Walter, D-56564 Neuwied (DE)
(74) Vertreter: Flaccus, Rolf-Dieter, Dr.
(86) Internationale Anmeldenummer: EP9602734
(87) Internationale Veröffentlichungsnummer: WO9702100

(56) Entgegenhaltungen:
- WO-A-92/09413
- US-A- 5 288 408

## Beschreibung

Die Erfindung betrifft ein Verfahren zur Entsorgung von Sondermüll in Form von Produktions-, Lager- und Gebrauchsabfällen von Wirkstoffe, insbesondere Arzneistoffe, enthaltenden Anordnungen für die transdermale Wirkstoffapplikation.

Vorrichtungen zur transdermalen Applikation können in zwei Kategorien unterteilt werden:
a. Systeme, die den Wirkstoff durch passive Diffusion an die Haut bzw. den Organismus abgeben und
b. Systeme, die die Wirkstoffe unter der Wirkung bzw. mit Unterstützung von elektrischen Strömen an die Haut bzw. den Organismus abgeben.

Systeme, die auf passiver Diffusion beruhen, sind die sogenannten Transdermalen Therapeutischen Systeme (TTS).
Sie können weiter unterteilt werden in die sogenannten Matrix- und Reservoirsysteme.
In den Matrixsystemen ist der Wirkstoff in Polymerfilmen gelöst oder zu einem gewissen Teil zusätzlich kristallin oder in Form von Mikrokapseln suspendiert. Solche Systeme bestehen deshalb im einfachsten Fall aus einer für den Wirkstoff im wesentlichen undurchlässigen Rückschicht, der wirkstoffhaltigen und bevorzugt selbstklebenden Matrix und einer vor Gebrauch zu entfernenden Schutzfolie.

Reservoirsysteme enthalten den Wirkstoff in einem flüssigen Reservoir. Er kann dabei komplett oder nur zum Teil gelöst vorliegen. Im einfachsten Fall bestehen solche Systeme aus einer für die sich im Reservoir befindenden Substanzen undurchlässigen Rückschicht und einer zumindest für den Wirkstoff durchlässigen Membran, die bevorzugt noch mit einem Kleberfilm zur Befestigung des Systems auf der Haut ausgestattet ist.

Systeme, die den Wirkstoff unter Anwendung von elektrischem Strom an die Haut bzw. den Organismus abgeben, können sehr unterschiedlich aufgebaut sein. Sie werden besonders eingesetzt für Wirkstoffe, die aufgrund ihrer chemisch-physikalischen Eigenschaften nicht in genügender Menge durch passive Diffusion die Haut durchdringen können.
Aus bekannten Gründen verbleibt in jedem getragenen TTS ein nicht verbrauchter Anteil Wirkstoff.
Im Bereich der TTS-Technik ergeben sich mithin erhebliche Entsorgungsmengen in Form von Produktionsabfällen, die bei bestimmten Produktionsverfahren zwangsläufig anfallen bzw. durch nicht immer vermeidbare Fehlchargen gebildet werden, (überalterten) Lagerbeständen und Gebrauchsresten, die insgesamt nur relativ geringe, aber hoch-toxische Mengen an Wirkstoff neben viel Ballast enthalten. Solche wirkstoffhaltigen Abfälle bedeuten ein toxikologisches bzw. ökologisches Risiko und sind daher als Sondermüll mit äußerst hohen Kosten zu entsorgen.

Aus dem Stand der Technik ist eine Vielzahl von Recyclingbzw. Entsorgungs-Verfahren bekannt, die sich mit der mengenmäßigen Hauptkomponente des Abfalls befassen, wie z.B. mit Abfällen von klebstoffbeschichtetem Folienmaterial. So beschreibt die DE 42 09 676 ein Recycling-Verfahren von drucksensitivem Selbstklebematerial mit einem erhaben ausgebildeten Abzugsfilm, insbesondere aus PET-Folienmaterial, laminiert mit EthylenTerephthalat-Isophthalatcopolyester.
DE 42 21 681 beschreibt ein Recycling-Verfahren von Polyethylen, Polypropylen oder Polystyrolklebern auf Etikettenabfällen.
DE 40 37 562 beschreibt ein Recycling-Verfahren für klebstoffbeschichtete Plastikfilme durch wiederholtes Kneten in Lösemittel, Abtropfenlassen in einem Schneckenförderer und mehrfaches Passieren verbleibenden Materials durch die gleiche Prozedur. Im einzelnen werden die mit Klebstoff beschichteten und in Schnitzelform vorliegenden KunststoffFolien in einer ersten Stufe in einem Lösungsmittel für den Klebstoff zum Dispergieren des an den Folienschnitzeln haftenden Klebstoffes mit dem Lösungsmittel für eine vorbestimmte Zeitdauer unter mechanischer Einwirkung bewegt. Danach erfolgt ein Trennen des im Lösungsmittel dispergierten Klebstoffs von den Folienschnitzeln durch mechanische Krafteinwirkung. Den aus der ersten Stufe aufgetragenen Folienschnitzeln wird in einer zweiten, gleichartigen Stufe frisches Lösungsmittel zugeführt und die Lösungsmittel-Kleberdispersion der zweiten Stufe wird der ersten Stufe zugeführt.

Bei einem aus der US-PS 5 288 408 bekannten Verfahren wird ein Kapsel- oder Rülsenmaterial auf Gelatinebasis zur Rückgewinnung von Gelatine und Weichmacher mit entionisiertem Wasser unter Bildung einer sog. "Bodenphase" behandelt, aus der eine reine konzentrierte wäßrige Gelatine-Glycerin-Lösung gewonnen wird. Den übrigen Abfallbestandteilen wie Vitaminen, Farbstoffen, Präservativen, die in einer oberen Ölphase anfallen, wird keine besondere Aufmerksamkeit geschenkt und lediglich erwähnt, daß deren mögliche Rezyclierung durch die Entfernung von Gelatine und Glycerin effizienter sein kann.

Die vorliegende Erfindung betrifft demgegenüber ein Entsorgungsverfahren, bei dem der im "Abfall" nur in relativ geringer Menge enthaltene Wirkstoff, insbesondere Arzneistoff, durch geeignete Extraktion vom restlichen Ballast so weit abgetrennt wird, daß für diesen eine teure Sonderentsorgung entfällt, während die durch Extraktion angereicherte Wirkstoffmenge, aus dem Extrakt rückgewonnen, einen für das Gesamtverfahren ökonomischen Wertposten darstellt.

Gemäß der Erfindung wird mithin ein Verfahren vorgesehen, mit dem es in wirtschaftlich vertretbarer Weise gelingt, Abfälle medizinischer Zubereitungen und insbesondere ungebrauchte bzw. nach dem Tragen abgelegte Vorrichtungen zur transdermalen Applikation von Wirkstoffen, sogenannte TTS, unter Rückgewinnung darin enthaltener Wirkstoffe aufzubereiten und zugleich die Entsorgung des Trägermaterials nach Entzug seines Wirkstoffinhaltes kostengünstig zu gestalten.

Das demgemäß entwickelte Verfahren der eingangs genannten Art ist Anspruch 1 zu entnahmens.

Das vorgeschlagene Verfahren ist umweltfreundlich, mit wirtschaftlichen Mitteln durchführbar und ergibt einerseits die Gewinnung eines wertvollen Rohwirkstoffes und andererseits eine von Inhaltsstoffen weitgehend befreite, umweltneutrale und daher problemlos entsorgbare Fraktion von Feststoffen bzw. Trägerstoffen.
Die Trägermaterialien bzw. anderen Rückstände aus dem Verfahren können nach aus dem Stand der Technik bekannten Methoden weiter recyclet werden.

Eine Ausgestaltung des Verfahrens sieht vor, daß die aufzubereitenden Materialien einer Vorbehandlung unterzogen und dabei, bezogen auf den Wirkstoff, bevorzugt in sortenreine Fraktionen getrennt, von Fremdstoffen wie Träger- und/oder Schutzfolien, Verpackungsmaterial etc. aussortiert und die wirkstoffhaltige Substanz konzentriert wird.
Eine weitere Ausgestaltung der Erfindung sieht vor, daß die der Wiederaufbereitung zuzuführenden Materialien (auch als "Abfälle" bezeichnet) in Verpackungen bzw. Verpackungshilfen verpackt der Recyclingstation angeliefert werden, und daß die Verpackungen bzw. Verpackungshilfen eine deutliche inhaltsidentische Kennzeichnung beispielsweise in Form eines Farbmusters oder eines für automatische Sortierung geeigneten Codierungsfeldes aufweisen. Mit Hilfe eines derartigen Codierungsfeldes könnte beispielsweise nach dem Vorbild einer Sortierung von Postgut eine Sortierung der codierten Verpackung in sortenreine Inhaltsstoffe vollautomatisch durchgeführt werden. Dadurch entfällt teure Handarbeit und die Sortierung wird fehlerlos durchführbar. Dies ist für ein Verfahren zur Gewinnung von Wirkstoffen aus beispielsweise TTS-Abfällen eminent wichtig. Zur Unterstützung der Lösung der wirkstoffhaltigen Substanz in einem Lösemittel kann es von Vorteil sein, daß die Abfälle einer Zerkleinerung fallweise unter Versprödung durch Kälte unterzogen werden. Unter anderem hat die Tiefkühlung mit flüssigen Stickstoff den weiteren Vorteil, daß so gekühlte Abfälle bei der Zerkleinerung keine schädlichen Stoffe an die Umwelt abgeben. Durch eine dabei erreichbare Zerkleinerung in Partikelgrößen beispielsweise unter 2 mm läßt sich das zerkleinerte Gut infolge Vergrößerung seiner aktiven Oberfläche im Lösungsmittelbad leichter und auch vollständiger lösen, bzw. der Wirkstoff durch Mazeration, Perkulation oder Wirbelschichtextraktion gewinnen.
Weiter sieht eine erfindungswesentliche Maßnahme vor, daß zum Lösen der wirkstoffhaltigen Substanz ein Lösemittel bzw. Lösemittelgemisch verwendet wird, welches ein vergleichsweise geringes Lösevermögen für Kleber und ein bevorzugtes Lösevermögen für den Wirkstoff aufweist. Die weitere Isolierung des Wirkstoffs aus der Lösung ist dann möglich durch z.B. Flüssig-Flüssig-Extraktion, Gegenstrom-Extraktion oder andere geeignete Verfahren.
Für die Gewinnung des Wirkstoffs aus der Lösung kann weiterhin vorgesehen sein, diese durch Fällen oder chemische Intermediär-Bindung oder Abscheiden von Fremdverbindungen Eindampfen, Destillieren oder andere geeignete Trennverfahren durchzuführen.

Bei der Extraktion der Wirkstoffe mit Lösemittel bzw. Lösemittelgemischen bietet es sich an, daß für basische Wirkstoffe Wasser mit einem sauren pH bzw. eine sauer eingestellte Wasser/Alkohol-Mischung verwendet wird und umgekehrt für saure Wirkstoffe ein basischer pH-Wert eingestellt wird.
Für Wirkstoffe ohne saure oder basische funktionelle Gruppen bietet es sich an, neutrale Wasser/Alkohol-Mischungen zu verwenden. Aus diesen Mischungen können die Wirkstoffe dann durch Änderung des pH-Wertes oder Zugabe von Wasser bzw. durch Flüssig/Flüssig-Extraktion entfernt werden. Die Verwendung von weitgehend wässrigen Lösemitteln hat darüber hinaus noch den Vorteil, daß weitgehend lipophile Hilfsstoffe nur zu einem geringen Teil mitextrahiert werden. Auch überkritische Gase können als Lösemittel zur Extraktion des Wirkstoffs eingesetzt werden. Ihr besonderer Vorteil ist, daß ihr Lösevermögen Temperatur und Druck und die Beimischung von zusätzlichen Lösemitteln in gewissen Grenzen beeinflußt werden kann und die extrahierten Bestandteile sehr leicht vom Lösemittel getrennt werden können. Da überkritische Gase über eine sehr geringe Viskosität verfügen und deshalb sehr schnell in das zu extrahierende Gut hineindiffundieren können, sind sie besonders geeignet, Wirkstoffe aus polymeren Materialien zu extrahieren. Wegen der guten Umweltverträglichkeit, des niedrigen Preises und der guten Handhabbarkeit eignet sich besonders überkritisches Kohlendioxid zu diesem Zweck. Weil nach Entzug von Wirkstoffen die Feststoffe dann weitestgehend von toxikologisch oder ökologisch risikohaften Stoffen befreit sind, können die so abgetrennten Feststoffe als umweltneutrale Abfälle problemlos beispielsweise durch Verbrennen oder Deponieren entsorgt werden. Die hierbei gegenüber den Deponiekosten von Sondermüll ersparten Kosten übersteigen die Kosten des erfindungsgemäßen Rückgewinnungsverfahrens. Darin steckt die wirtschaftliche Chance für ein Wirkstoffrückgewinnungsverfahren. Die in das Lösungsmittel in Lösung übergegangenen Wirk- und Inhaltsstoffe lassen sich aus der Lösung bzw. aus den Lösemittelgemischen durch bekannte thermische Reinigungsverfahren wie Destillation bzw. Rektifikation in an sich bekannter und bewährter Weise mit vergleichsweise geringem Aufwand an Kosten und Energien zurückgewinnen.

Weiterhin ist vorgesehen, daß der aus der Lösung gewonnene Roh-Wirkstoff in einem finalen Arbeitsschritt zu pharmazeutisch reinem Wirkstoff aufbereitet wird.

Die dazu geeigneten Verfahren sind dem Fachmann bekannt, beispielhaft seien genannt Umkristallisation oder präparative Hoch- bzw. Mitteldruckchromatographie.

Das Verfahren ist zweckmäßig und wirtschaftlich vorteilhaft und erfüllt in optimaler Weise die eingangs gestellte Aufgabe.
Durch Wahl der Verfahrensparameter können insbesondere Wirkstoffe mit hormonartiger Wirkung, Estradiol, Estradiolderivate, Gestagene, Gestagenderivate oder ihre Gemische, Morphin- oder Morphinderivate, Buprenorphin, Physostigmin, Scopolamin, Galanthamin wiedergewonnen werden. Die wiedergewonnenen Stoffe können dann erneut je nach ihrer pharmazeutischen Wirkung als Schmerzmittel sowie zur Behandlung seniler Demenz, von Bluthochdruck, Arrhythmien, Gefäßkrankheiten, Suchtkrankheiten, erhöhten Blutfettspiegeln, seelischen Störungen, zur Beeinflußung der Blutgerinnung, von Eßstörungen oder von Störungen des Zuckerstoffwechsels verwendet werden.

## Patentansprüche

1. Entsorgungsverfahren mit Rückgewinnung von Wirkstoffen aus ungebrauchten bzw. abgelegten Vorrichtungen zur transdermalen Applikation von Wirkstoffen (TTS) und/oder deren produktionsabfällen, wobei die ungebrauchten Vorrichtungen bzw. Abfälle als wirkstoffhaltige Substanz eine fallweise haftklebende Matrix oder ein Reservoir enthalten, worin der Wirkstoff in untergeordneter Menge als homogenes Gemisch, in Form von Mikrokapseln oder Kristallen oder als flüssige Lösung enthalten ist, bzw. in Verbindung mit Polymerfilmen und polymerfolien vorliegen, dadurch gekennzeichnet, daß die ungebrauchten bzw. abgelegten TTS bzw. die Abfälle der Entsorgungsstation in Verpackungen bzw. Verpackungshilfen verpackt angeliefert werden, die eine deutliche inhaltsidentische Kennzeichnung aufweisen, und daß der oder die Wirkstoff(e) und gegebenenfalls weitere Bestandteile in einem selektiven Lösungsmittel in Lösung gebracht und aus dieser der Wirkstoff wiedergewonnen wird, während die ausreichend wirkstoff-verarmte Restmasse einer insbesondere konventionellen Abfallbeseitigung oder Rezyklierung zugeführt wird.

2. Verfahren nach Anspruch 1, dadurch gekennzeichnet, daß die Kennzeichnung in einem Farbmuster oder einem für automatische Sortierung geeigneten Codierungsfeld besteht.

3. Verfahren nach Anspruch 1 oder 2, dadurch gekennzeichnet, daß
die ungebrauchten bzw. abgelegten TTS bzw. die Abfälle einer Vorbehandlung unterzogen und dabei in sortenreine Fraktionen getrennt von Fremdstoffen, Träger- und/oder Schutzfolien, Verpackungsmaterial aussortiert und die wirkstoffhaltige Substanz konzentriert wird.

4. Verfahren nach einem oder mehreren der Ansprüche 1 bis 3, dadurch gekennzeichnet, daß die ungebrauchten bzw. abgelegten TTS bzw. die Abfälle einer Zerkleinerung fallweise unter Versprödung durch Kälte, unterzogen werden.

5. Verfahren nach einem oder mehreren der Ansprüche 1 bis 4, dadurch gekennzeichnet, daß zum Lösen des Wirkstoffe oder der Wirkstoffe ein Lösemittel bzw. ein Lösemittelgemisch verwendet wird, welches ein vergleichsweise geringes Lösevermögen für Kleber und andere Bestandteile des TTS und ein bevorzugtes Lösevermögen für den Wirkstoff oder die Wirkstoffe aufweist.

6. Verfahren nach einem oder mehreren der Ansprüche 1 bis 5, dadurch gekennzeichnet, daß das Lösen der Substanz unter intensivem Rühren und fallweise bei erhöhter Temperatur durchgeführt wird.

7. Verfahren nach einem oder mehreren der Ansprüche 1 bis 6, dadurch gekennzeichnet, daß die Extraktion des Wirkstoffs aus der Lösung durch
- Flüssig-Flüssig-Extraktion
- Mazeration oder Perkolation
- Gegenstrom-Extraktion
- Wirbelschicht-Extraktion
oder andere geeignete Verfahren durchgeführt wird.

8. Verfahren nach einem oder mehreren der Ansprüche 1 bis 7, dadürch gekennzeichnet, daß die Gewinnung des Wirkstoffes aus der Lösung durch Fällen, chemische Intermediärbindung, Abscheiden von Fremdverbindungen, Eindampfen, Destillieren oder andere geeignete Trennverfahren durchgeführt wird.

9. Verfahren nach Anspruch 5, dadurch gekennzeichnet, daß für das Lösen von basischen Wirkstoffen Wasser mit saurem pH bzw. eine sauer eingestellte Wasser/Alkoholmischung verwendet wird.

10. Verfahren nach Anspruch 5, dadurch gekennzeichnet, daß für das Lösen der Wirkstoffe neutrale oder basisch eingestellte Wasser/Alkoholmischungen verwendet werden.

11. Verfahren nach einem der Ansprüche 1 bis 4, dadurch gekennzeichnet, daß zum Lösen des Wirkstoffe bzw. der Wirkstoffe überkritische Gase, vorzugsweise Kohlendioxid, verwendet werden.

12. Verfahren nach einem der Ansprüche 1 bis 11, dadurch gekennzeichnet, daß der aus der Lösung gewonnene Wirkstoff bzw. die Wirkstoffe in einem finalen Arbeitsschritt zu pharmazeutisch reinem Wirkstoff aufgearbeitet wird.

13. Verfahren nach einem oder mehreren der Ansprüche 1 bis 11, dadurch gekennzeichnet, daß die Rektifikation der Rohwirkstoffe durch
- destillative Trennverfahren, oder durch
- Hochdruckextraktion mit überkritischen Gasen, bevorzugt CO₂, erfolgt.

14. Verfahren nach einem oder mehreren der Ansprüche 1 bis 12, dadurch gekennzeichnet, daß aus den Wasser/Alkohol-Wirkstoffmischungen die Wirkstoffe durch Änderung des pH-Wertes oder Zugabe von Wasser bzw. durch Flüssig-Flüssig-Extraktion entfernt werden.

## Claims

1. Disposal process with recovery of active substances from unused or discarded devices for the transdermal application of active substances (TTS) and/or their process waste, the unused devices or waste products having as active substance-containing material either a matrix, which optionally may be pressure-sensitive adhesive, or a reservoir, wherein the active substance is comprised in small amount as a homogeneous mixture, in the form of microcapsules or crystals, or as a liquid solution, or being present in combination with polymeric films and polymeric sheets, characterized in that the unused or discarded TTSs or waste products are delivered to the disposal station packed in packages or packaging aids which have a distinct, content-indicating identification, and that the active substance(s) and optionally further components is/are brought into solution in a selective solvent, and that the active substance is recovered therefrom, whereas the sufficiently active substance-depleted residual mass is supplied to a waste disposal or recycling process, especially a conventional one.

2. The process according to claim 1 characterized in that the identification is in the form of a color pattern or a coded field suitable for automatic sorting.

3. The process according to claim 1 or 2 characterized in that the unused or discarded TTSs or the waste products are subjected to a pretreatment sorting them into pure-grade fractions which have been separated from foreign matter, such as supporting and/or protective sheets, packaging material, and that the active substance-containing material is concentrated.

4. The process according to one or several of claims 1 to 3 characterized in that the unused or discarded TTSs or the waste products are subjected to a size reduction, optionally under low-temperature embrittlement.

5. The process according to one or several of claims 1 to 4 characterized in that a solvent or solvent mixture is used to dissolve the active substance or active substances, which has a comparatively low dissolving capacity for adhesives and other components of the TTS and a preferred solubilizing power for the active substance/s.

6. The process according to one or several of claims 1 to 5 characterized in that the dissolution of the substance is carried out under intensive stirring and optionally at an elevated temperature.

7. The process according to one or several of claims 1 to 6 characterized in that the extraction of the active substance from the solution is carried out by means of
- liquid-liquid extraction
- maceration or percolation
- countercurrent extraction
- fluidized-bed extraction
or other suitable methods.

8. The process according to one or several of claims 1 to 7 characterized in that the recovery of the active substance from the solution is carried out by means of precipitation, chemical intermediary bonding, deposition of foreign compounds, evaporation, distillation, or other suitable separation methods.

9. The process according to claim 5 characterized in that water with an acidic pH or an acidified water/alcohol mixture is used to dissolve basic active substances.

10. The process according to claim 5 characterized in that neutral or basified water/alcohol mixtures are used to dissolve the active substances.

11. The process according to any one of claims 1 to 4 characterized in that supercritical gases, preferably carbon dioxide, are used to dissolve the active substance or active substances.

12. The process according to any one of claims 1 to 11 characterized in that the active substance/s recovered from the solution is/are reprocessed to pharmaceutically pure active substance in a final operation.

13. The process according to one or several of claims 1 to 11 characterized in that the rectification of the raw active substances is effected by
- separation methods by distillation, or by
- high-pressure extraction with supercritical gases, preferably CO₂.

14. The process according to one or several of claims 1 to 12 characterized in that the active substances are removed from the water/alcohol/active substance mixtures by changing the pH-value or by adding water, or by means of liquid-liquid extraction.

## Revendications

1. Procédé d'élimination avec récupération des substances actives de dispositifs inutilisés resp. enlevés pour l'application transdermique de substances actives (STT) et/ou leurs déchets de production, les dispositifs inutilisés ou les déchets contenant, comme substance contenant la substance active, une matrice, le cas échéant adhésive, ou un réservoir dans lequel la substance active est contenue en une quantité inférieure en tant que mélange homogène, sous forme de microcapsules ou de cristaux, ou en tant que solution liquide, resp. se trouve en association avec des films et des feuilles de polymère, caractérisé en ce que les STT inutilisés resp. enlevés resp. les déchets provenant de la station d'élimination sont livrés emballés dans des emballages resp. des accessoires d'emballage, qui présentent une caractérisation nette d'identification du contenu et en ce que la ou les substances actives et, le cas échéant, d'autres composants sont dissous dans un solvant sélectif dans une solution et en ce qu'on récupère la substance active à partir de celle-ci, alors que la masse résiduelle suffisamment appauvrie en substance active est alimentée dans un processus, en particulier conventionnel, d'élimination des déchets ou de recyclage.

2. Procédé selon la revendication 1, caractérisé en ce que la caractérisation consiste en une marque de couleur ou un champ de codification approprié pour le tri automatique.

3. Procédé selon la revendication 1 ou 2, caractérisé en ce que le STT inutilisé resp. enlevé ou les déchets sont soumis à un prétraitement et triés en des fractions sélectives selon le type, séparées des substances étrangères, films de support et/ou de protection et matériau d'emballage et en ce que la substance contenant la substance active est concentrée.

4. Procédé selon une ou plusieurs des revendications 1 à 3, caractérisé en ce que le STT inutilisé resp. enlevé ou les déchets sont soumis à un broyage, le cas échéant à une fragilisation par le froid.

5. Procédé selon une ou plusieurs des revendications 1 à 4, caractérisé en ce qu'on utilise, pour la dissolution de la substance active ou des substances actives, un solvant resp. un mélange de solvants qui présente un pouvoir de dissolution relativement faible pour la colle et les autres constituants du STT et un pouvoir de dissolution préféré pour la substance active ou les substances actives.

6. Procédé selon une ou plusieurs des revendications 1 à 5, caractérisé en ce que la dissolution de la substance est réalisée sous agitation intense et, le cas échéant, à température plus élevée.

7. Procédé selon une ou plusieurs des revendications 1 à 6, caractérisé en ce que l'extraction de la substance active de la solution est réalisée par
- extraction liquide-liquide
- macération ou percolation
- extraction à contre-courant
- extraction en lit fluidisé
ou par d'autres procédés appropriés.

8. Procédé selon une ou plusieurs des revendications 1 à 7, caractérisé en ce que l'extraction de la substance active de la solution est réalisée par précipitation, fixation chimique intermédiaire, séparation des composés étrangers, concentration par évaporation, distillation ou par d'autres procédés de séparation appropriés.

9. Procédé selon la revendication 5, caractérisé en ce qu'on utilise, pour la dissolution des substances actives basiques, de l'eau avec un pH acide resp. un mélange eau/ alcool réglé dans une plage acide.

10. Procédé selon la revendication 5, caractérisé en ce qu'on utilise, pour la dissolution des substances actives, des mélanges eau/ alcool neutres ou réglés dans une plage basique.

11. Procédé selon une quelconque des revendications 1 à 4, caractérisé en ce qu'on utilise, pour la dissolution de la substance active resp. des substances actives, des gaz surcritiques, de préférence du dioxyde de carbone.

12. Procédé selon une quelconque des revendications 1 à 11, caractérisé en ce que la substance active resp. les substances actives, extraites à partir de la solution, sont transformées dans une étape de travail finale en substance active pharmaceutiquement pure.

13. Procédé selon une ou plusieurs des revendications 1 à 11, caractérisé en ce que la rectification des substances actives brutes est réalisée par
- des procédés de séparation par distillation ou par
- extraction sous pression élevée, à l'aide de gaz surcritiques, de préférence du CO₂.

14. Procédé selon une ou plusieurs des revendications 1 à 12, caractérisé en ce qu'on élimine les substances actives des mélanges eau/ alcoolsubstance active par modification du pH ou par addition d'eau resp. par une extraction liquide-liquide.
